# EUROPEAN PATENT SPECIFICATION

(11) **EP 3 344 633 B1**
(45) Date of publication and mention of the grant of the patent: **03.07.2019**
(21) Application number: 15763989.9
(22) Date of filing: 03.09.2015
(51) Int. Cl.: C07F 3/02, C07F 5/06, H01L 51/00

(54) **CARBON DIOXIDE SENSING COMPOUNDS**
KOHLENDIOXIDERFASSUNGSVERBINDUNGEN
COMPOSÉS DE DÉTECTION DE DIOXYDE DE CARBONE

(43) Date of publication of application: 11.07.2018
(73) Proprietor: Robert Bosch GmbH, 70442 Stuttgart (DE)
(72) Inventor: YAN, Yan, Singapore 573943 (SG); PALALE, Suresh, Singapore 573943 (SG); LOHANI, Anup, Singapore 573943 (SG)
(86) International application number: PCT/SG2015/050297
(87) International publication number: WO 2017/039528

(56) References cited:
- CN-A- 101 196 489
- CN-B- 102 692 445

## Description

### Technical Field

The invention relates to carbon dioxide sensing compounds. In particular, present invention relates to said sensing compounds comprising a metal phthalocycanine. Furthermore, the invention relates to the tuning sensitivity of the metal phthalocyanine by incorporation of amine groups and spacers.

### Background

Carbon dioxide (CO₂) is a normal constituent of exhaled breath, and is commonly measured as an indicator to evaluate whether adequate fresh outdoor air are being introduced into indoor air. If indoor CO₂ levels are more than 1,000 ppm, there is probably inadequate ventilation. Complaints such as headaches, fatigue, and eye and throat irritation may then become prevalent. CO₂ on its own is not responsible for the complaints; however, a high level of CO₂ may indicate that other contaminants in the building are also present at elevated levels and most likely to be responsible for occupant complaints.

At even higher level, CO₂ can cause asphyxiation as it replaces oxygen in the blood, so exposure to concentrations around 40,000 ppm is immediately dangerous to life and health. Therefore, more work is required concerning CO₂ sensing and sensor development for indoor air quality control (IAQ).

To this end, several materials has been proposed as a CO₂ sensing layer. In one example, metal oxide semiconductors, such as BaSnO₃, TiO₂, CuO-SnO₂, and perovskite, have been widely studied for CO₂ sensing due to their low cost and simple preparation methods. However, these materials offen suffer from high power consumption and low selectivity. Although carbon nanotube and graphene-metal-oxide composite can be operated in relatively lower temperature, the selectivity is still unsolved.

There are some reported organic polymer layers for CO₂ detection, for example, polysiloxane, polythiophene, polypyrrole, polyethyleneimine, and polyaniline. However, the unsatisfying sensitivity, selectivity and life time impede most of them as appropriate candidates as CO₂ sensing layers. Some sensing layers with small organic dye molecules integrated therein can also respond to CO₂ indirectly by sensing a change in the pH. This method is mainly useful for optical senisng only, and shows inadequate sensitivity as well as excessive moisture interference.

Metal phthalocyanines (MPc), a versatile aromatic macrocycles, has been extensively studied and identified as a promising candidate for gas sensors. Compared to polymers, MPc shows less moisture interference and better tunability (i.e. various options and combinations for the central cavity M and substitution groups). Additionally, MPc has good processability, i.e. it can be easily processed in either evaporation or solution processing, while polymers can only be processed from solution. There is an increasing market for CO₂ sensor in consumer electronics (CE) for IAQ. In one case, reflow soldering is a necessary process in production of CE, which requires higher thermal stability of sensing layer (able to withstand up to 260°C heating in ambient air). The poor thermal stability of polymers thus hinder them as candidates in such applications.
The documents CN 102 692 445 B and CN 101 196 489 A disclose metal phthalocyanines for the use as gas sensors.

Therefore, there remains a need to provide for alternative carbon dioxide sensing compounds that overcome, or at least alleviate, the above problems.

### Summary

Present inventors have herein identified phthalocyanine, a versatile aromatic macrocycle, as a promising carbon dioxide sensing compound. Compared to other organic macrocyclic compounds, phthalocyanine has good processability, thermal stability, tunability, and high selectivity achievable by tuning its central cavity and substitution groups. In particular, amines are able to react with CO₂ and therefore affords a feasbile means for detecting/sensing
CO₂. Accordingly, present inventors have made use of this sensing capability and proposed incorporating amine groups into the MPc. Present inventors have further proposed to insert a spacer moiety to the amine groups.

Thus, in accordance with one aspect of the invention, there is provided a use of a compound of Formula (I) 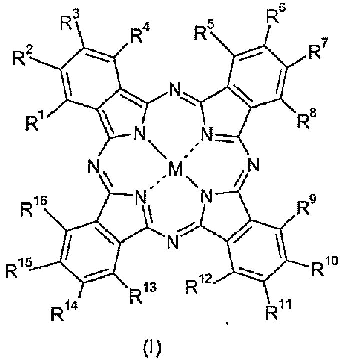 as a carbon dioxide sensor,
wherein:
M is any suitable metallic species; and
each of R¹ to R¹⁶ is independently a H or an organic moiety of Formula (II)

   -X-Y-NR¹⁷R¹⁸ (II),

   wherein:
   X is a direct bond or a heteroatom selected from the group consisting of N, O, P, S, and Se;
   Y is a linear or branched, substituted or unsubstituted C₁-C₁₀ alkyl; linear or branched, substituted or unsubstituted C₂-C₁₀ alkenyl; linear or branched, substituted or unsubstituted C₂-C₁₀ alkynyl; linear or branched, substituted or unsubstituted C₁-C₁₀ alkoxy; substituted or unsubstituted C₃-C₁₀ cycloalkyl; substituted or unsubstituted C₃-C₁₀ cycloalkenyl; substituted or unsubstituted C₆-C₁₀ aryl; substituted or unsubstituted C₃-C₁₀ heteroaryl; and
   each of R¹⁷ and R¹⁸ is a H or a linear or branched, substituted or unsubstituted C₁-C₁₀ alkyl;
with the proviso that not all of R¹ to R¹⁶ are H.

In various embodiments, use of the compound of Formula (I) may include use as a sensing layer in various transducers like a chemiresistor, a capacitor, a field effect transistor (FET), an optical-based sensor, or a mass-based sensor.

In another aspect of the invention, a sensor for detecting carbon dioxide is disclosed. The sensor comprises a compound of Formula (I) 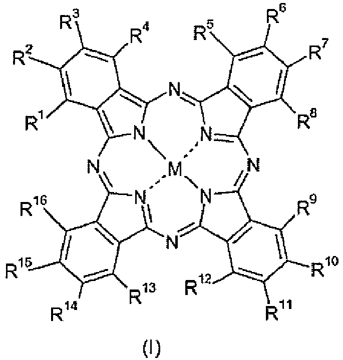 wherein:
M is any suitable metallic species; and
each of R¹ to R¹⁶ is independently a H or an organic moiety of Formula (II)

   -X-Y-NR¹⁷R¹⁸ (II),

   wherein:
   X is a direct bond or a heteroatom selected from the group consisting of N, O, P, S, and Se;
   Y is a linear or branched, substituted or unsubstituted C₁-C₁₀ alkyl; linear or branched, substituted or unsubstituted C₂-C₁₀ alkenyl; linear or branched, substituted or unsubstituted C₂-C₁₀ alkynyl; linear or branched, substituted or unsubstituted C₁-C₁₀ alkoxy; substituted or unsubstituted C₃-C₁₀ cycloalkyl; substituted or unsubstituted C₃-C₁₀ cycloalkenyl; substituted or unsubstituted C₆-C₁₀ aryl; substituted or unsubstituted C₃-C₁₀ heteroaryl; and
   each of R¹⁷ and R¹⁸ is a H or a linear or branched, substituted or unsubstituted C₁-C₁₀ alkyl;
with the proviso that not all of R¹ to R¹⁶ are H.

### Brief Description of the Drawings

In the drawings, like reference characters generally refer to the same parts throughout the different views. The drawings are not necessarily drawn to scale, emphasis instead generally being placed upon illustrating the principles of various embodiments. In the following description, various embodiments of the invention are described with reference to the following drawings.
**Fig. 1** shows two different reaction paths of CO₂ with primary amine (R is H) and secondary amine group (R is not H).
**Fig. 2** illustrates the Lewis interaction between an amine-functionalized MPc and CO₂.
**Fig. 3** illustrates the present amine-spacer-MPc concept.
**FIG. 4** shows various amine-functionalized MPc sensing layers illustrated in the example section.
**Fig. 5** shows the response comparison among various sensing layers to CO₂ from 400 to 2,000 ppm for the various amine-functionaiized MPc sensing layers of **Fig. 4****.** For the Layer 1 sensing layer, no response to CO₂ was detected.

### Description

The following detailed description refers to the accompanying drawings that show, by way of illustration, specific details and embodiments in which the invention may be practised. These embodiments are described in sufficient detail to enable those skilled in the art to practise the invention. Other embodiments may be utilized and chemical or structural changes may be made without departing from the scope of the invention. The various embodiments are not necessarily mutually exclusive, as some embodiments can be combined with one or more other embodiments to form new embodiments.

As described in previous paragraphs, metal phthalocyanine (MPc) demonstrates various advantages over other organic compounds as a carbon dioxide sensing material. For example, metal phthalocyanine has good processability, thermal stability, tunability, and high selectivity achievable by tuning its central cavity and more particularly, its substitution groups.

Accordingly, it is herein described a metal phthalocyanine chemical platform for selective and sensitive CO₂ sensing. Utilizing the large design space of the MPc layer, the sensitivity of MPc to CO₂ can be easily enhanced by modifying its substitution groups and the respective substitution number. Taking together the mentioned advantages of MPc over other materials, functionalized MPc is a good candidate for CO₂ sensing layer. Changes caused by CO₂ interaction can be measured by means of a workfunction, capacitance, mass, absorption wavelengths etc. such that the resultant sensing layer is compatible with various transducers including but not limited to a chemiresistor, a capacitor, a field effect transistor (FET), an optical-based sensor, or a mass-based sensor.

Additionally, functionalized MPc can react directly and reversibly with CO₂ via the side chains, i.e. the substitution groups, which leads to higher sensitivity and selectivity. Furthermore, MPc offers a large material design space by affording the ability to change the functional substitution groups, which translates to a superior tunability.

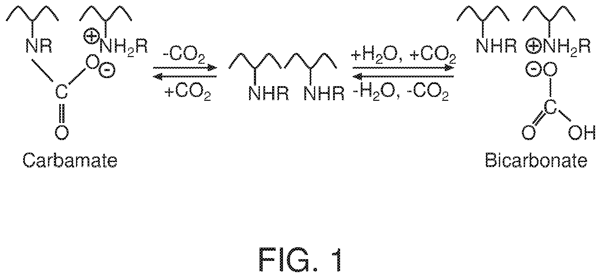

It is known that primary amine group (-NH₂) and secondary amine group (-NHR) react with CO₂ in two different ways (**Fig. 1**). On one hand, one amine group can form one bicarbonate in the presence of water. On the other hand, two amine groups can form a carbamate complex without water. The former is effective at higher temperature, while the latter starts at lower temperature, e.g. room temperature (RT).

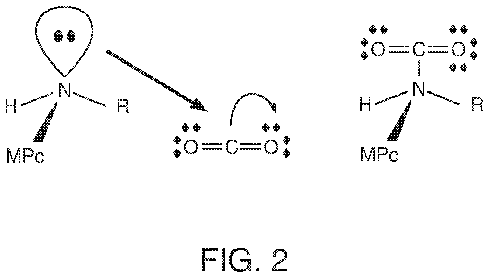

The nature of the reaction generating carbamate is Lewis interaction (**Fig. 2**), i.e. CO₂ is a Lewis acid (LA) whereas the amine group is a Lewis base (LS). The nature of the reaction yielding bicarbonate is acid-base reaction, i.e. CO₂ is an acid and the amine group is a base. For both reactions, the stronger the basicity of the amine group is, the stronger is the interaction with CO₂ and hence, the CO₂ sensitivity of the compound. Briefly, Lewis acid is a species that accepts an electron pair and has vacant orbitals. Lewis base is a species that donates an electron pair and has lone pair of electrons. When a Lewis base donates its lone pair of electrons to a Lewis acid, an acid-base complex or complex ion is formed.

The interaction, and therefore detection, of carbon dioxide and the present metal phthalocyanine sensing compound is based on the reaction between the carbon dioxide and the amine groups substituted on the metal phthalocyanine.

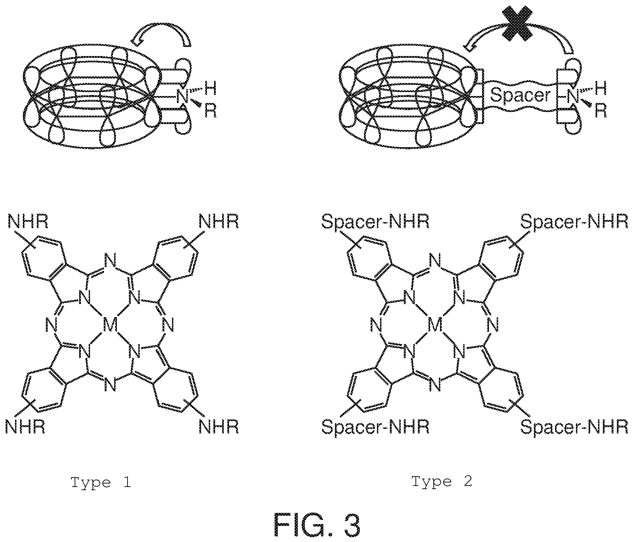

A stronger basicity in the amine group is preferred for the CO₂ sensing. This thus means that any interaction which reduces the basicity of the amine group (i.e. electron density on nitrogen atom) is likely to sacrifice on the sensitivity to CO₂ and should be avoided. As an illustration, a simple amine-functionalized MPc is one with amine groups directly linked to the MPc (Type 1 in **Fig. 3**). However, such compound has been found to show very weak or no response to CO₂ (i.e. below the detection limit of the detecting device) due to the conjugation between the aromatic ring and the amine group. In this case, the amine group donates its electron to the MPc aromatic system and reduces its electron density and hence basicity. To increase the interaction with CO₂, a spacer moiety is deliberately introduced to the amine group to minimize the conjugation effect (Type 2 in **Fig. 3**).

Based on the above design parameters, present invention therefore relates to a use of a compound of Formula (I) 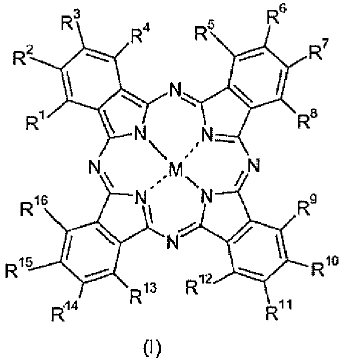 as a carbon dioxide sensor,
wherein:
M is any suitable metallic species; and
each of R¹ to R¹⁶ is independently a H or an organic moiety of Formula (II)

   -X-Y-NR¹⁷R¹⁸ (II),

   wherein:
   X is a direct bond or a heteroatom selected from the group consisting of N, O, P, S, and Se;
   Y is a linear or branched, substituted or unsubstituted C₁-C₁₀ alkyl; linear or branched, substituted or unsubstituted C₂-C₁₀ alkenyl; linear or branched, substituted or unsubstituted C₂-C₁₀ alkynyl; linear or branched, substituted or unsubstituted C₁-C₁₀ alkoxy; substituted or unsubstituted C₃-C₁₀ cycloalkyl; substituted or unsubstituted C₃-C₁₀ cycloalkenyl; substituted or unsubstituted C₆-C₁₀ aryl; substituted or unsubstituted C₃-C₁₀ heteroaryl; and
   each of R¹⁷ and R¹⁸ is a H or a linear or branched, substituted or unsubstituted C₁-C₁₀ alkyl;
   with the proviso that not all of R¹ to R¹⁶ are H.

Accordingly, the spacer moiety in the chemical moiety of Formula (II) is -X-Y-.

In present context, the term "aliphatic", alone or in combination, refers to a straight chain (i.e. linear) or branched chain hydrocarbon comprising at least one carbon atom. Aliphatics include alkyls, alkenyls, and alkynyls. In certain embodiments, aliphatics are optionally substituted, i.e. substituted or unsubstituted. The term "optionally substituted" or "substituted or unsubstituted" refers to a group in which none, one, or more than one of the hydrogen atoms have been replaced with one or more groups such as, but are not limited to, alkyl, heteroalkyl, haloalkyl, heteroholoalkyl, cycloalkyl, aryl, arylalkyl, heteroaryl, or non-aromatic heterocycle.

Aliphatics include, but are not limited to, methyl, ethyl, propyl, isopropyl, butyl, isobutyl, tert-butyl, pentyl, hexyl, ethenyl, propenyl, butenyl, ethynyl, butynyl, propynyl, and the like, each of which may be optionally substituted. As used herein, aliphatic is not intended to include cyclic groups.

In present context, the term "alkyl", alone or in combination, refers to a fully saturated aliphatic hydrocarbon. The alkyl may be linear or branched. In certain embodiments, alkyls are optionally substituted. In certain embodiments, an alkyl comprises 1 to 10 carbon atoms, for example 1 to 5 carbon atoms, wherein (whenever it appears herein in any of the definitions given below) a numerical range, such as "1 to 10" or "C₁-C₁₀", refers to each integer in the given range, e.g. "C₁-C₁₀ alkyl" means that an alkyl group comprising only 1 carbon atom, 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms. Examples of alkyl groups include, but are not limited to, methyl, ethyl, n-propyl, isopropyl, n-butyl, isobutyl, sec-butyl, tert-butyl, tert-amyl, pentyl, hexyl, heptyl, octyl and the like.

In present context, the term "alkoxy", alone or in combination, refers to an aliphatic hydrocarbon having an alkyl-O- moiety. The alkoxy may be linear or branched. In certain embodiments, alkoxy groups are optionally substituted. In various embodiments, the alkoxy comprises 1 to 10 carbon atoms, i.e. C₁-C₁₀ alkoxy. Examples of alkoxy groups include, but are not limited to, methoxy, ethoxy, propoxy, butoxy and the like.

In present context, the term "alkenyl", alone or in combination, refers to an aliphatic hydrocarbon having one or more carbon-carbon double-bonds, such as two or three carbon-carbon double-bonds. The alkenyl may be linear or branched. In certain embodiments, alkenyls are optionally substituted, i.e. substituted or unsubstituted. In certain embodiments, an alkenyl comprises 2 to 10 carbon atoms. "C₂-C₁₀ alkenyl" means that an alkenyl group comprising only 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms. Examples of alkenyls include, but are not limited to, ethenyl, propenyl, butenyl, 1,4-butadienyl, pentenyl, hexenyl, 4-methylhex-1-enyl, 4-ethyl-2-methylhex-1-enyl and the like.

In present context, the term "alkynyl", alone or in combination, refers to an aliphatic hydrocarbon having one or more carbon-carbon triple-bonds, such as two or three carbon-carbon triple-bonds. The alkynyl may be linear or branched. In certain embodiments, alkynyls are optionally substituted, i.e. substituted or unsubstituted. In certain embodiments, an alkynyl comprises 2 to 10 carbon atoms. "C₂-C₁₀ alkynyl" means that an alkynyl group comprising only 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms. Examples of alkynyls include, but are not limited to, ethynyl, propynyl, butynyl, and the like.

In present context, the term "non-aromatic ring" refers to a group comprising a covalently closed ring that is not aromatic. The term "alicyclic" refers to a group comprising a non-aromatic ring wherein each of the atoms forming the ring is a carbon atom. Alicyclic groups may be formed by three, four, five, six, seven, eight, nine, or more than nine carbon atoms. In certain embodiments, alicyclics are optionally substituted, i.e. substituted or unsubstituted. In certain embodiments, an alicyclic comprises one or more unsaturated bonds, such as one or more carbon-carbon double-bonds. Alicyclics include cycloalkyls and cycloalkenyls. In various embodiments, R₂ may be a substituted or unsubstituted C₃-C₁₀ cycloalkyl. In further embodiments, R₂ may be a substituted or unsubstituted C₃-C₁₀ cycloalkenyl. Examples of alicyclics include, but are not limited to, cyclopropane, cyclobutane, cyclopentane, cyclopentene, cyclopentadiene, cyclohexane, cyclohexene, 1,3-cyclohexadiene, 1,4-cyclohexadiene, cycloheptane, and cycloheptene.

In present context, the term "aryl" refers to an aromatic ring wherein each of the atoms forming the ring is a carbon atom. Aryl rings may be formed by five, six, seven, eight, nine, or more than nine carbon atoms. Aryl groups may be optionally substituted.

In present context, the term "heteroaryl" refers to an aromatic heterocycle. Heteroaryl rings may be formed by three, four, five, six, seven, eight, nine, or more than nine atoms. Heteroaryls may be optionally substituted. Examples of heteroaryl groups include, but are not limited to, aromatic C₃-C₈ heterocyclic groups comprising one oxygen or sulfur atom or up to four nitrogen atoms, or a combination of one oxygen or sulfur atom and up to two nitrogen atoms, and their substituted as well as benzo- and pyrido-fused derivatives, for example, connected via one of the ring-forming carbon atoms.

As mentioned in earlier paragraphs, M may be any suitable metallic species that form the MPc. For example, M may be, but is not limited to, selected from the group consisting of Cu²⁺, Mn²⁺, Mg²⁺, Ca²⁺, B³⁺, Fe³⁺, Al³⁺, Ga³⁺, In³⁺, Ce³⁺, Sc³⁺, Zr⁴⁺, Ti⁴⁺, Sn⁴⁺, and V⁵⁺.

In various embodiments, M may be Cu²⁺, Mn²⁺, Mg²⁺, or Ca²⁺.

In preferred embodiments, M is Cu²⁺.

In various embodiments, two or more of R¹ to R¹⁶ are each the organic moiety of Formula (II). In other words, at most one of R¹ to R¹⁶ is a H.

Since the carbamate complex formation requires two amine groups to be present (**Fig**. 1), adjoining amines allow both reaction paths (i.e. carbamate and bicarbonate) to occur. Accordingly, adjoining-amine-spacer substitution is introduced into the MPc to further enhance its sensitivity to CO₂.

Thus, in preferred embodiments, at least the pair of R² and R³, or the pair of R⁶ and R⁷, or the pair of R¹⁰ and R¹¹, or the pair of R¹⁴ and R¹⁵ is the organic moiety of Formula (II).

More preferably, all of R², R³, R⁶, R⁷, R¹⁰, R¹¹, R¹⁴, and R¹⁵ are each the organic moiety of Formula (II) whereas all of R¹, R⁴, R⁵, R⁸, R⁹, R¹², R¹³, and R¹⁶ are H.

In certain embodiments, in the organic moiety of Formula (II):
X is an O;
Y is a C₁-C₁₀ alkyl, C₃-C₁₀ cycloalkyl, or C₆-C₁₀ aryl; and
both R¹⁷ and R¹⁸ are H.

Preferably, the organic moiety of Formula (II) is -O-(CH₂)ₙNH₂, wherein n is an integer of between 1 and 10.

In preferred embodiments, the compound of Formula (I) is: 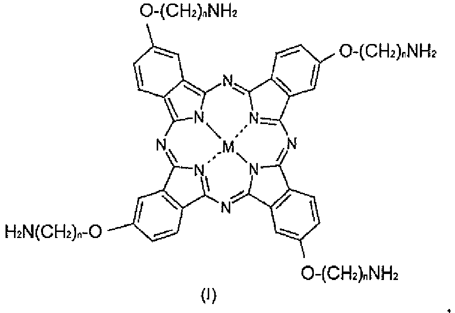 where n is an integer of between 1 and 10, preferably between 1 and 8, more preferably between 1 and 6.

More preferably, the compound of Formula (I) is: 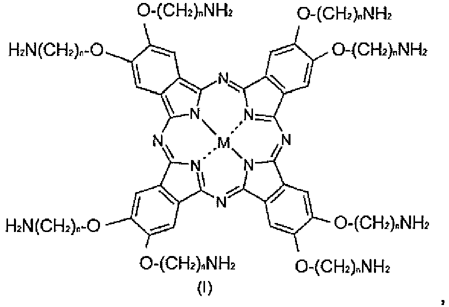 where n is an integer of between 1 and 10, preferably between 1 and 8, more preferably between 1 and 6.

In various embodiments, use of the compound of Formula (I) may incude use as a sensing layer in various transducers like a chemiresistor, a capacitor, a field effect transistor (FET), an optical-based sensor, or a mass-based sensor.

In another aspect of the invention, a sensor for detecting carbon dioxide is disclosed. The sensor comprises a compound of Formula (I) 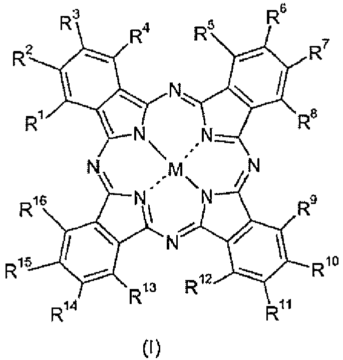 wherein:
M is any suitable metallic species; and
each of R¹ to R¹⁶ is independently a H or an organic moiety of Formula (II)

   -X-Y-NR¹⁷R¹⁸ (II),

   wherein:
   X is a direct bond or a heteroatom selected from the group consisting of N, O, P, S, and Se;
   Y is a linear or branched, substituted or unsubstituted C₁-C₁₀ alkyl; linear or branched, substituted or unsubstituted C₂-C₁₀ alkenyl; linear or branched, substituted or unsubstituted C₂-C₁₀ alkynyl; linear or branched, substituted or unsubstituted C₁-C₁₀ alkoxy; substituted or unsubstituted C₃-C₁₀ cycloalkyl; substituted or unsubstituted C₃-C₁₀ cycloalkenyl; substituted or unsubstituted C₆-C₁₀ aryl; substituted or unsubstituted C₃-C₁₀ heteroaryl; and
   each of R¹⁷ and R¹⁸ is a H or a linear or branched, substituted or unsubstituted C₁-C₁₀ alkyl;
with the proviso that not all of R¹ to R¹⁶ are H.

In order that the invention may be readily understood and put into practical effect, particular embodiments will now be described by way of the following non-limiting examples.

### Example

In this example, the adjoining-amine-spacer-MPc concept is illustrated, where n is between 1 and 6.

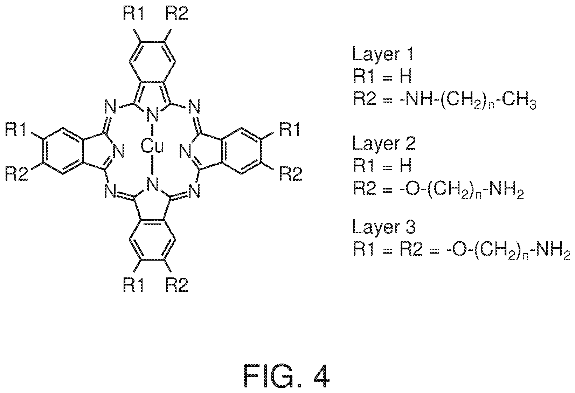

Three amine-functionalized MPc sensing layers are prepared (**Fig. 4**). Layer 1 has no spacer between the MPc and amine group. Layer 2 is an amine-spacer functionalized MPc. Layer 3 has adjoining-amine-spacer substitution.

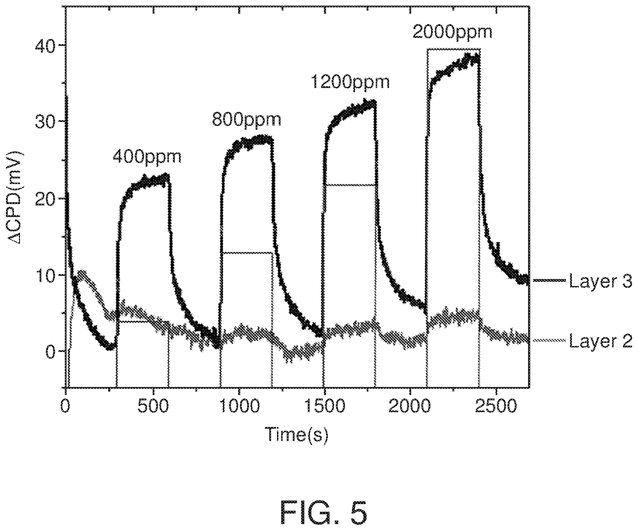

To read out the change of work function, the Kelvin method was used. Gas measurement was carried out with synthetic air at RT with 50% RH (relative humidity) for CO₂ concentration from 400 ppm (background in atmosphere) up to 2,000 ppm. As shown in **Fig. 5****,** the work function delivered a distinct and reversible response to CO₂ (400 ppm up to 2,000 ppm) using Layer 3, and the sensitivity was higher than Layer 2, whereas for Layer 1, no response to CO₂ was detected.

By "comprising" it is meant including, but not limited to, whatever follows the word "comprising". Thus, use of the term "comprising" indicates that the listed elements are required or mandatory, but that other elements are optional and may or may not be present.

By "consisting of" is meant including, and limited to, whatever follows the phrase "consisting of". Thus, the phrase "consisting of" indicates that the listed elements are required or mandatory, and that no other elements may be present.
The inventions illustratively described herein may suitably be practiced in the absence of any element or elements, limitation or limitations, not specifically disclosed herein. Thus, for example, the terms "comprising", "including", "containing", etc. shall be read expansively and without limitation.

By "about" in relation to a given numerical value, such as for temperature and period of time, it is meant to include numerical values within 10% of the specified value.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

## Claims

1. Use of a compound of Formula (I) 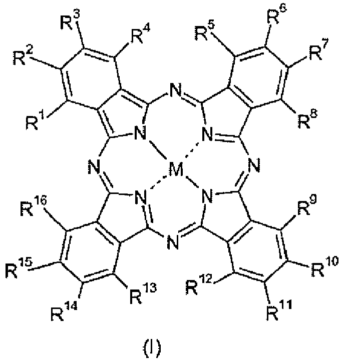 as a carbon dioxide sensor,
wherein:
M is any suitable metallic species; and
each of R¹ to R¹⁶ is independently a H or an organic moiety of Formula (II)
-X-Y-NR¹⁷R¹⁸ (II),
wherein:
X is a direct bond or a heteroatom selected from the group consisting of N, O, P, S, and Se;
Y is a linear or branched, substituted or unsubstituted C₁-C₁₀ alkyl; linear or branched, substituted or unsubstituted C₂-C₁₀ alkenyl; linear or branched, substituted or unsubstituted C₂-C₁₀ alkynyl; linear or branched, substituted or unsubstituted C₁-C₁₀ alkoxy; substituted or unsubstituted C₃-C₁₀ cycloalkyl; substituted or unsubstituted C₃-C₁₀ cycloalkenyl; substituted or unsubstituted C₆-C₁₀ aryl; substituted or unsubstituted C₃-C₁₀ heteroaryl; and
each of R¹⁷ and R¹⁸ is a H or a linear or branched, substituted or unsubstituted C₁-C₁₀ alkyl;
with the proviso that not all of R¹ to R¹⁶ are H.

2. Use of claim 1, wherein M is selected from the group consisting of Cu²⁺, Mn²⁺, Mg²⁺, Ca²⁺, B³⁺, Fe³⁺, Al³⁺, Ga³⁺, In³⁺, Ce³⁺, Sc³⁺, Zr⁴⁺, Ti⁴⁺, Sn⁴⁺, and V⁵⁺.

3. Use of claim 1, wherein M is Cu²⁺.

4. Use of claim 1, wherein two or more of R¹ to R¹⁶ are each the organic moiety of Formula (II).

5. Use of claim 1, wherein R², R³, R⁶, R⁷, R¹⁰, R¹¹, R¹⁴, and R¹⁵ are each the organic moiety of Formula (II).

6. Use of claim 1, wherein in the organic moiety of Formula (II),
X is an O;
Y is a C₁-C₁₀ alkyl, C₃-C₁₀ cycloalkyl, or C₆-C₁₀ aryl; and
both R¹⁷ and R¹⁸ are H.

7. Use of claim 1, wherein the organic moiety of Formula (II) is -O-(CH₂)ₙNH₂, wherein n is an integer of between 1 and 10.

8. Use of claim 1, wherein the compound of Formula (I) is 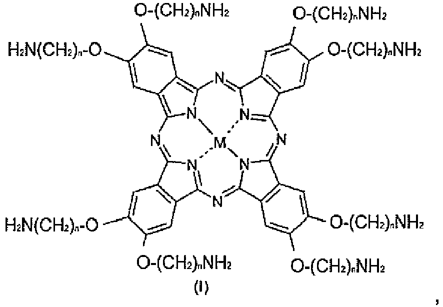 wherein n is an integer of between 1 and 10.

9. Use of claim 1, wherein the compound of Formula (I) is 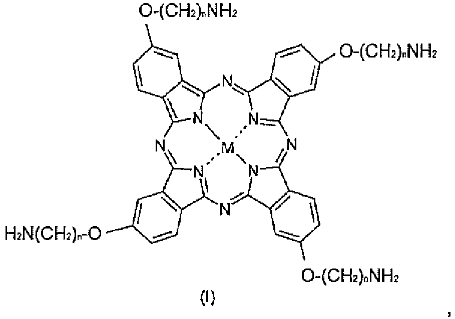 wherein n is an integer of between 1 and 10.

10. Use of claim 1, wherein the compound of Formula (I) is comprised as a sensing layer in a chemiresistor, a capacitor, a field effect transistor (FET), an optical-based sensor, or a mass-based sensor.

11. A sensor for detecting carbon dioxide, the sensor comprising a compound of Formula (I) 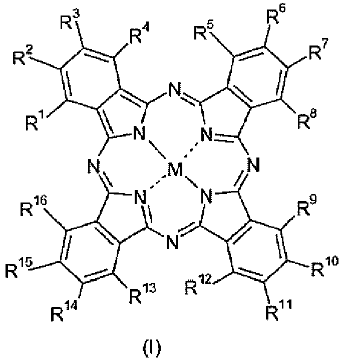 wherein:
M is any suitable metallic species; and
each of R¹ to R¹⁶ is independently a H or an organic moiety of Formula (II)
-X-Y-NR¹⁷R¹⁸ (II),
wherein:
X is a direct bond or a heteroatom selected from the group consisting of N, O, P, S, and Se;
Y is a linear or branched, substituted or unsubstituted C₁-C₁₀ alkyl; linear or branched, substituted or unsubstituted C₂-C₁₀ alkenyl; linear or branched, substituted or unsubstituted C₂-C₁₀ alkynyl; linear or branched, substituted or unsubstituted C₁-C₁₀ alkoxy; substituted or unsubstituted C₃-C₁₀ cycloalkyl; substituted or unsubstituted C₃-C₁₀ cycloalkenyl; substituted or unsubstituted C₆-C₁₀ aryl; substituted or unsubstituted C₃-C₁₀ heteroaryl; and
each of R¹⁷ and R¹⁸ is a H or a linear or branched, substituted or unsubstituted C₁-C₁₀ alkyl;
with the proviso that not all of R¹ to R¹⁶ are H.

## Patentansprüche

1. Verwendung einer Verbindung der Formel (I) 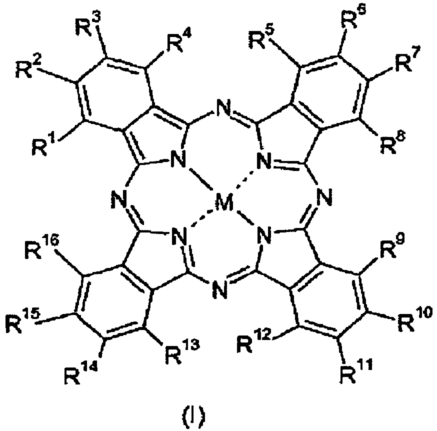 als Kohlendioxidsensor,
wobei:
M für eine beliebige geeignete Metallspezies steht; und
R¹ bis R¹⁶ jeweils unabhängig für ein H oder eine organische Gruppierung der Formel (II)
-X-Y-NR¹⁷R¹⁸ (II)
stehen,
wobei:
X für eine direkte Bindung oder ein Heteroatom aus der Gruppe bestehend aus N, O, P, S und Se steht;
Y für ein lineares oder verzweigtes, substituiertes oder unsubstituiertes C₁-C₁₀-Alkyl; lineares oder verzweigtes, substituiertes oder unsubstituiertes C₂-C₁₀-Alkenyl; lineares oder verzweigtes, substituiertes oder unsubstituiertes C₂-C₁₀-Alkinyl; lineares oder verzweigtes, substituiertes oder unsubstituiertes C₁-C₁₀-Alkoxy; substituiertes oder unsubstituiertes C₃-C₁₀-Cycloalkyl; substituiertes oder unsubstituiertes C₃-C₁₀-Cycloalkenyl; substituiertes oder unsubstituiertes C₆-C₁₀-Aryl; substituiertes oder unsubstituiertes C₃-C₁₀-Heteroaryl steht; und
R¹⁷ und R¹⁸ jeweils für ein H oder ein lineares oder verzweigtes, substituiertes oder unsubstituiertes C₁-C₁₀-Alkyl stehen;
mit der Maßgabe, dass nicht alle Variablen R¹ bis R¹⁶ für H stehen.

2. Verwendung nach Anspruch 1, wobei M aus der Gruppe bestehend aus Cu²⁺, Mn²⁺, Mg²⁺, Ca²⁺, B³⁺, Fe³⁺, Al³⁺, Ga³⁺, In³⁺, Ce³⁺, Sc³⁺, Zr⁴⁺, Ti⁴⁺, Sn⁴⁺ und V⁵⁺ ausgewählt ist.

3. Verwendung nach Anspruch 1, wobei M für Cu²⁺ steht.

4. Verwendung nach Anspruch 1, wobei zwei oder mehr von R¹ bis R¹⁶ jeweils für die organische Gruppierung der Formel (II) stehen.

5. Verwendung nach Anspruch 1, wobei R², R³, R⁶, R⁷, R¹⁰, R¹¹, R¹⁴ und R¹⁵ jeweils für die organische Gruppierung der Formel (II) stehen.

6. Verwendung nach Anspruch 1, wobei in der organischen Gruppierung der Formel (II)
X für ein O steht;
Y für ein C₁-C₁₀-Alkyl, C₃-C₁₀-Cycloalkyl oder C₆-C₁₀-Aryl steht; und
sowohl R¹⁷ als auch R¹⁸ für H stehen.

7. Verwendung nach Anspruch 1, wobei es sich bei der organischen Gruppierung der Formel (II) um -O-(CH₂)ₙNH₂ handelt, wobei n für eine ganze Zahl zwischen 1 und 10 steht.

8. Verwendung nach Anspruch 1, wobei es sich bei der Verbindung der Formel (I) um 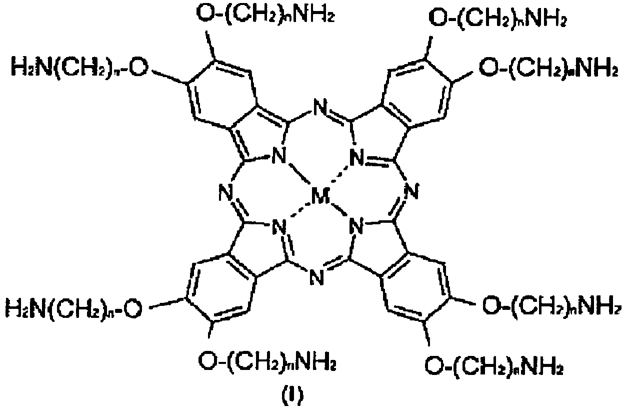 handelt, wobei n für eine ganze Zahl zwischen 1 und 10 steht.

9. Verwendung nach Anspruch 1, wobei es sich bei der Verbindung der Formel (I) um 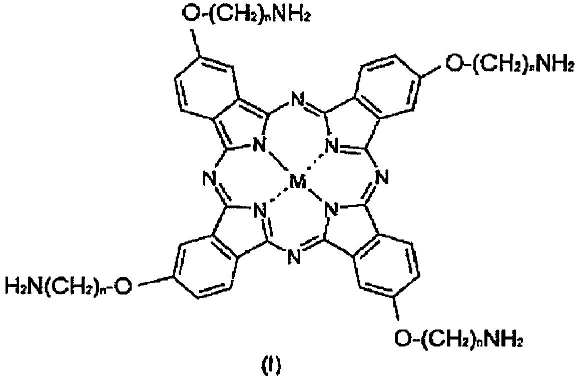 handelt, wobei n für eine ganze Zahl zwischen 1 und 10 steht.

10. Verwendung nach Anspruch 1, wobei die Verbindung der Formel (I) als Sensorschicht in einem Chemiresistor, einem Kondensator, einem Feldeffekttransistor (FET), einem optikbasierten Sensor oder einem massebasierten Sensor enthalten ist.

11. Sensor zum Nachweis von Kohlendioxid, wobei der Sensor eine Verbindung der Formel (I) 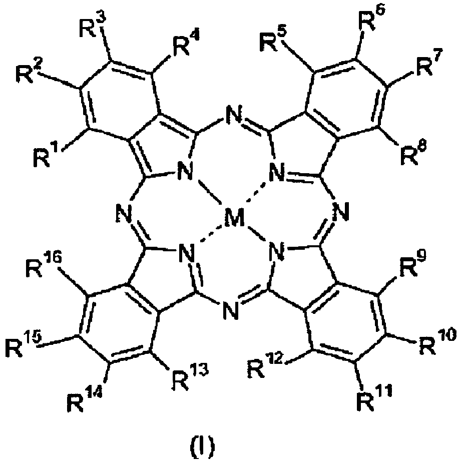 umfasst, wobei:
M für eine beliebige geeignete Metallspezies steht; und
R¹ bis R¹⁶ jeweils unabhängig für ein H oder eine organische Gruppierung der Formel (II)
-X-Y-NR¹⁷R¹⁸ (II)
stehen,
wobei:
X für eine direkte Bindung oder ein Heteroatom aus der Gruppe bestehend aus N, O, P, S und Se steht;
Y für ein lineares oder verzweigtes, substituiertes oder unsubstituiertes C₁-C₁₀-Alkyl; lineares oder verzweigtes, substituiertes oder unsubstituiertes C₂-C₁₀-Alkenyl; lineares oder verzweigtes, substituiertes oder unsubstituiertes C₂-C₁₀-Alkinyl; lineares oder verzweigtes, substituiertes oder unsubstituiertes C₁-C₁₀-Alkoxy; substituiertes oder unsubstituiertes C₃-C₁₀-Cycloalkyl; substituiertes oder unsubstituiertes C₃-C₁₀-Cycloalkenyl; substituiertes oder unsubstituiertes C₆-C₁₀-Aryl; substituiertes oder unsubstituiertes C₃-C₁₀-Heteroaryl steht; und
R¹⁷ und R¹⁸ jeweils für ein H oder ein lineares oder verzweigtes, substituiertes oder unsubstituiertes C₁-C₁₀-Alkyl stehen;
mit der Maßgabe, dass nicht alle Variablen R¹ bis R¹⁶ für H stehen.

## Revendications

1. Utilisation d'un composé de formule (I) 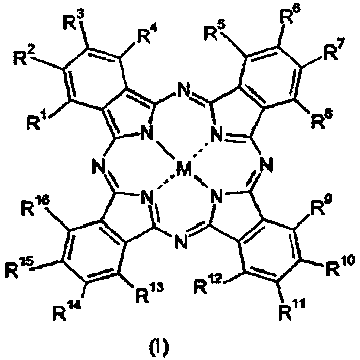 en tant que capteur de dioxyde de carbone,
dans lequel :
M est une espèce métallique adaptée ; et
chacun de R¹ à R¹⁶ est indépendamment H ou un fragment organique de formule (II)
-X-Y-NR¹⁷R¹⁸ (II),
dans lequel :
X est une liaison directe ou un hétéroatome choisi dans le groupe constitué de N, O, P, S et Se ;
Y est un alkyle en C₁-C₁₀ linéaire ou ramifié, substitué ou non substitué ; un alcényle en C₂-C₁₀ linéaire ou ramifié, substitué ou non substitué ; un alcynyle en C₂-C₁₀ linéaire ou ramifié, substitué ou non substitué ; un alcoxy en C₁-C₁₀ linéaire ou ramifié, substitué ou non substitué ; un cycloalkyle en C₃-C₁₀ substitué ou non substitué ; un cycloalcényle en C₃-C₁₀ substitué ou non substitué ; un aryle en C₆-C₁₀ substitué ou non substitué ; un hétéroaryle en C₃-C₁₀ substitué ou non substitué ; et
chacun de R¹⁷ et R¹⁸ est H ou un alkyle en C₁-C₁₀ linéaire ou ramifié, substitué ou non substitué ;
à condition que R¹ à R¹⁶ ne soient pas tous H.

2. Utilisation selon la revendication 1, dans laquelle M est choisi dans le groupe constitué de Cu²⁺, Mn²⁺, Mg²⁺, Ca²⁺, B³⁺, Fe³⁺, Al³⁺, Ga³⁺, ln³⁺, Ce³⁺, Sc³⁺, Zr⁴⁺, Ti⁴⁺, Sn⁴⁺ et V⁵⁺.

3. Utilisation selon la revendication 1, dans laquelle M est Cu²⁺.

4. Utilisation selon la revendication 1, dans laquelle deux ou plus de deux R¹ à R¹⁶ sont chacun le fragment organique de formule (II).

5. Utilisation selon la revendication 1, dans laquelle R², R³, R⁶, R⁷, R¹⁰, R¹¹, R¹⁴ et R¹⁵ sont chacun le fragment organique de formule (II).

6. Utilisation selon la revendication 1, dans laquelle, dans le fragment organique de formule (II),
X est O ;
Y est un alkyle en C₁-C₁₀, un cycloalkyle en C₃-C₁₀ ou un aryle en C₆-C₁₀ ; et
R¹⁷ et R¹⁸ sont tous deux H.

7. Utilisation selon la revendication 1, dans laquelle le fragment organique de formule (II) est -O-(CH₂)ₙNH₂, dans lequel n est un entier compris entre 1 et 10.

8. Utilisation selon la revendication 1, dans laquelle le composé de formule (I) est 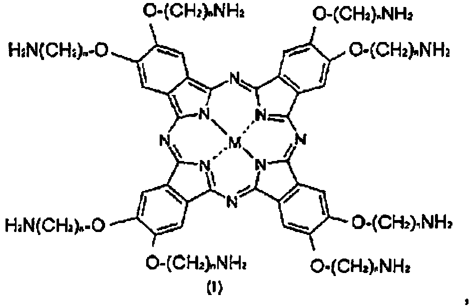 dans lequel n est un entier compris entre 1 et 10.

9. Utilisation selon la revendication 1, dans laquelle le composé de formule (I) est 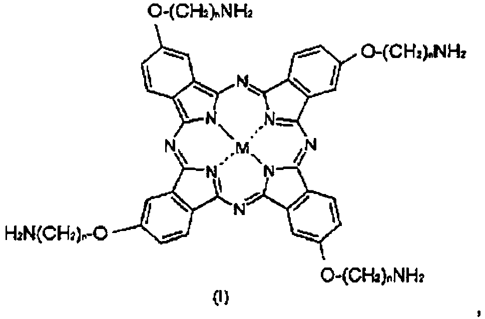 dans lequel n est un entier compris entre 1 et 10.

10. Utilisation selon la revendication 1, dans laquelle le composé de formule (I) est compris en tant que couche de détection dans une chimiorésistance as a sensing couche dans a chemiresistor, un condensateur, un transistor à effet de champ (TEF), un capteur à base d'optique ou un capteur à base masse.

11. Capteur pour détecter le dioxyde de carbone, le capteur comprenant un composé de formule (I) 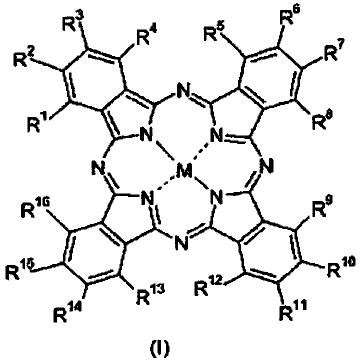 dans lequel :
M est une espèce métallique adaptée ; et
chacun de R¹ à R¹⁶ est indépendamment H ou un fragment organique de formule (II)
-X-Y-NR¹⁷R¹⁸ (II),
dans lequel :
X est une liaison directe ou un hétéroatome choisi dans le groupe constitué de N, 0, P, S et Se ;
Y est un alkyle en C₁-C₁₀ linéaire ou ramifié, substitué ou non substitué ; un alcényle en C₂-C₁₀ linéaire ou ramifié, substitué ou non substitué ; un alcynyle en C₂-C₁₀ linéaire ou ramifié, substitué ou non substitué ; un alcoxy en C₁-C₁₀ linéaire ou ramifié, substitué ou non substitué ; un cycloalkyle en C₃-C₁₀ substitué ou non substitué ; un cycloalcényle en C₃-C₁₀ substitué ou non substitué ; un aryle en C₆-C₁₀ substitué ou non substitué ; un hétéroaryle en C₃-C₁₀ substitué ou non substitué ; et
chacun de R¹⁷ et R¹⁸ est H ou un alkyle en C₁-C₁₀ linéaire ou ramifié, substitué ou non substitué ;
à condition que R¹ à R¹⁶ ne soient pas tous H.
